# EUROPEAN PATENT APPLICATION

(11) **EP 2 192 539 A1**
(43) Date of publication of application: **02.06.2010**
(21) Application number: 08170185.6
(22) Date of filing: 28.11.2008
(51) Int. Cl.: G06Q 10/00

(54) **Method of executing a task part of a clinical pathway in a computer implemented medical information system**

(71) Applicant: Agfa HealthCare AG, 54294 Trier (DE)
(72) Inventor: Penkaitis, Wilhelm, 2640, Mortsel (BE); Muys, Herman, Dr., 2640, Mortsel (BE); Baur, Thomas, Dr., 2640, Mortsel (BE)
(74) Representative: Verbrugghe, Anne Marie L.

(57) **Abstract**

Upon selection of a task part of a clinical pathway in a user interface of a computer implemented medical information system, a technical configuration for the task type is looked up in a task repository and a task executer is determined by its corresponding plug-in to execute the task.

## Description

### FIELD OF THE INVENTION

The present invention relates to computer implemented medical information system and more particularly relates to clinical pathways used for patient care.

### BACKGROUND OF THE INVENTION

A clinical pathway or care pathway is a guideline representing the actions to be taken for a specific group of patients, for instance patients with special complaints, a particular disease or having a certain procedure done.
Clinical pathways are used to describe and implement clinical standards. They help to provide quality and efficient patient care. Nowadays the tasks of a clinical pathway to be applied to a patient are represented as a hard copy task-time matrix with time on the horizontal axis and the tasks sorted by category organized in the vertical direction.
The table is commonly manually filed out and updated. This operation is rather cumbersome and does not permit a lot of flexibility.

In computer-implemented medical information systems a clinical pathway can be managed in a more convenient way.
However, even with computer-implemented clinical pathways some problems need to be solved. Among these problems is the fact that the implementation has to work in a heterogeneous environment and often is host-dependent.

It is an aspect of the present invention to provide a method for enhanced managing of clinical pathways in a computer-implemented information system.

### SUMMARY OF THE INVENTION

The above-mentioned aspects are realised by a method having the specific features set out in claim 1.

Specific features for preferred embodiments of the invention are set out in the dependent claims.

The present invention relates to a method of executing a task part of a clinical pathway in a computer implemented medical information system. Upon selection of the task in a user interface, a technical configuration for the task type is looked up in a task repository and a task executer is determined by its corresponding plug-in and the task is executed by the determined executer.

A task to be executed is typically the display of a form to be filled out by a doctor or nurse and to be distributed to the caregiver. Such a task will typically be an order request like e.g. a radiology examination, medication, ECG etc. but could also be a diet or physiotherapy prescription.
A task could as well be a non interactive HL7/IHE request handled (through connectivity) by third party.

The present invention is generally implemented as a computer program product adapted to carry out the method described below and may be stored on a computer readable medium.

Further advantages and embodiments of the present invention will become apparent from the following description and drawings.

### BRIEF DESCRIPTION OF THE DRAWINGS

Fig. 1 shows a screen print illustrating the lay out of the user interface of a clinical pathway application,

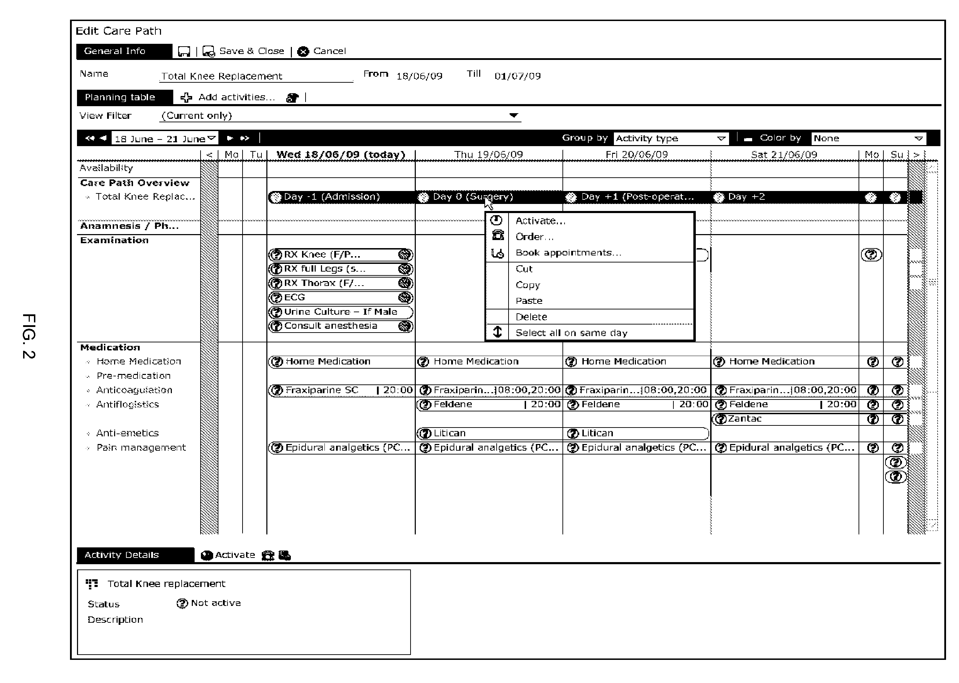

Fig. 2 and 3 are screen prints illustrating appointment booking,
Fig. 4 is a collaboration diagram illustrating a basic task execution.

### DETAILED DESCRIPTION OF THE INVENTION

### Lay-out

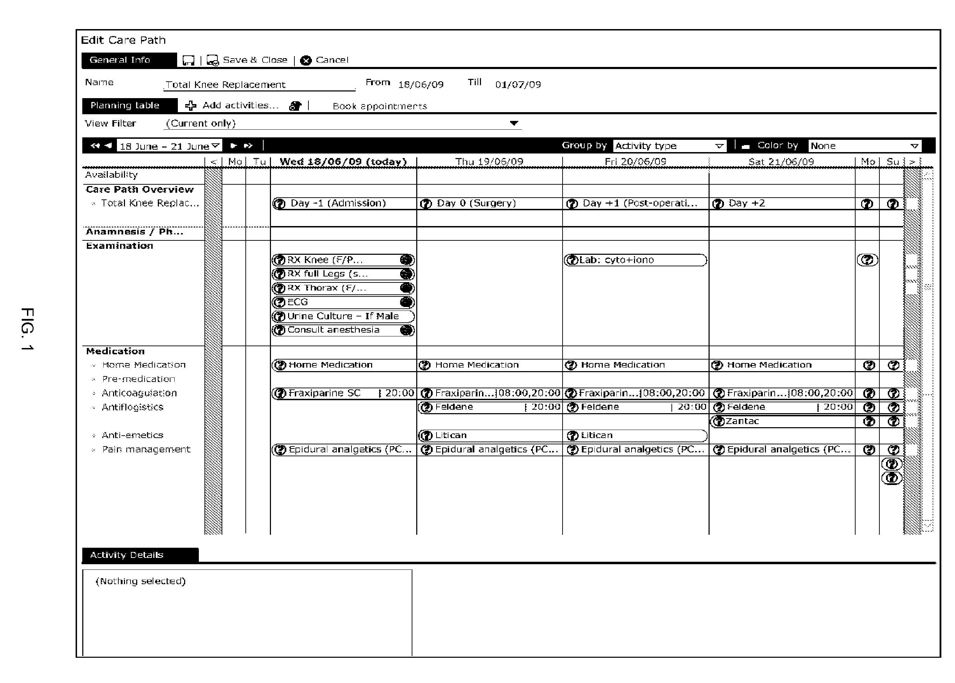

Figure 1 shows the lay-out of a user interface for clinical pathways.

A clinical pathway selected for a particular patient is displayed in a matrix (implemented in the form of a spread sheet) wherein time is represented on the horizontal axis and the tasks are organized in vertical direction. The tasks are sorted by category such as type of examination, medication etc. These tasks are for example in the case of medication: home medication, pre-medication etc.
The different tasks of a clinical pathway that are to be performed on successive days (can also be hours, months, other periods ...) are shown in the corresponding row and column.
Some tasks such as RX knee, RX full leg etc. have a button indicating that these task can be scheduled. The button is checked when the scheduling has been performed.

To schedule certain tasks the following actions are performed on the user interface and in a scheduling engine which is addressed by the medical information system executed by a host engine.

### Scheduling

Upon selection of a task that can be scheduled a request for scheduling an appointment is sent to a scheduling engine such as IPlan+ of Agfa HealthCare. The constraints pertaining to that action are also sent to the scheduling engine. For example in case of a RX of the knee, the constraints are that a radiology room has to be free and an operator and/or radiologist have to be available. Another type of constraints are constraints relating to the dependencies between tasks. For example, a radiograph is to be taken prior to surgery.

The scheduling engine calculates all possible appointments corresponding with the given constraints and sends the calculated available time stamps to the medical information system.

The operation of a scheduling engine has been described extensively in the published PCT application WO 2005/004013.

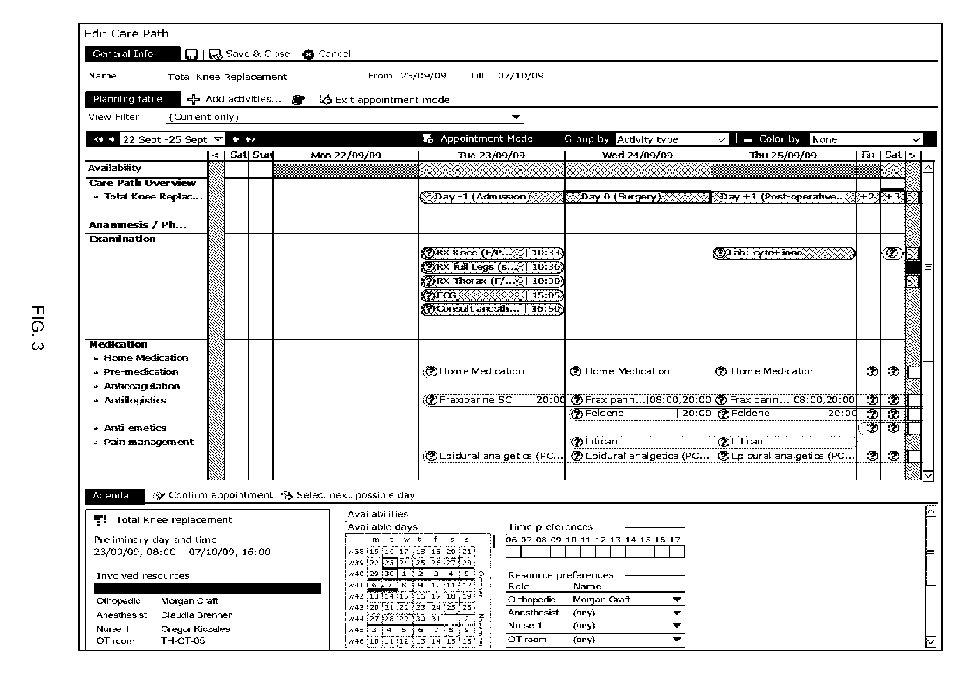

The possible appointment time stamps are then displayed, permitting the user to select an appropriate time stamp. Display of availabilities is shown at the bottom of figure 3. On a calendar different colors may be used to distinguish between available and occupied time stamps. Buttons to confirm an appointment or to select a next possible day may be provided.

Upon interaction of the user, a time stamp is selected and confirmed. The confirmation is fed back into the scheduling engine where the time stamp is labeled as being occupied.

It is also possible to postpone scheduling of a task in a pathway. Not all tasks need to be scheduled at creation of the pathway. For example in case 10 days after surgery a control radiography is to be taken, an icon can be displayed for example on the 7^{th} day after surgery reminding the user to schedule the control radiography.

### Move a task along the time axis

In the user interface the possibility exists to move a task to another day by dragging the task along the time axis (in the described implementation this task is shifted in the horizontal direction) to another location.
For scheduled tasks, the scheduling engine is then informed that the previously selected time slot is to be released and the scheduling operation is restarted with the new constraints.
The availabilities are again communicated from the scheduling engine to the medical information system and eventually displayed so that a new time stamp can be selected, communicated to the scheduling engine and confirmed.

If a task moves to another point in time, all dependent tasks are moved along and rescheduled.

### Independent resolving

The application allows so-called independent resolving which relates to the situation in which more than one clinical pathway is considered for a single patient.
The tasks of both care plans are displayed on the same user interface as described higher.
Independent resolving relates to the fact that shifts in the planning of tasks for one clinical pathway have no influence on the planning of tasks on the same days for the other clinical pathway for the same patient.
For example if in one care plan an RX is planned before surgery and the surgery task is moved to another day, the RX will shift together with the surgery and the relative position of RX and surgery will remain constant. This is achieved by a hierarchical, tree-like, temporal constraint model which is connected to every runtime instance of a clinical path based on a predefined template. Moving an element leads to a move of all child elements and a change of the constraint to the parent element. After the action all the element dates are recalculated.
However, tasks pertaining to another clinical pathway which is set up for the same patient (e.g. relating to diabetes) does not shift simultaneously with the shift of a task in the first pathway.
Due to the fact that every path instance holds it's own temporal model the shift inside one path instance does not influence other paths.

### Independency from host system

The implementation of the tasks of clinical pathways in a medical information system is set up to be independent from the type of host system.
For example suppose that an RX task is part of a clinical pathway. When this task is selected by the user in the user interface, a task type repository is addressed in which a configuration is selected corresponding with the selected task type but adapted for execution by the envisaged host system.

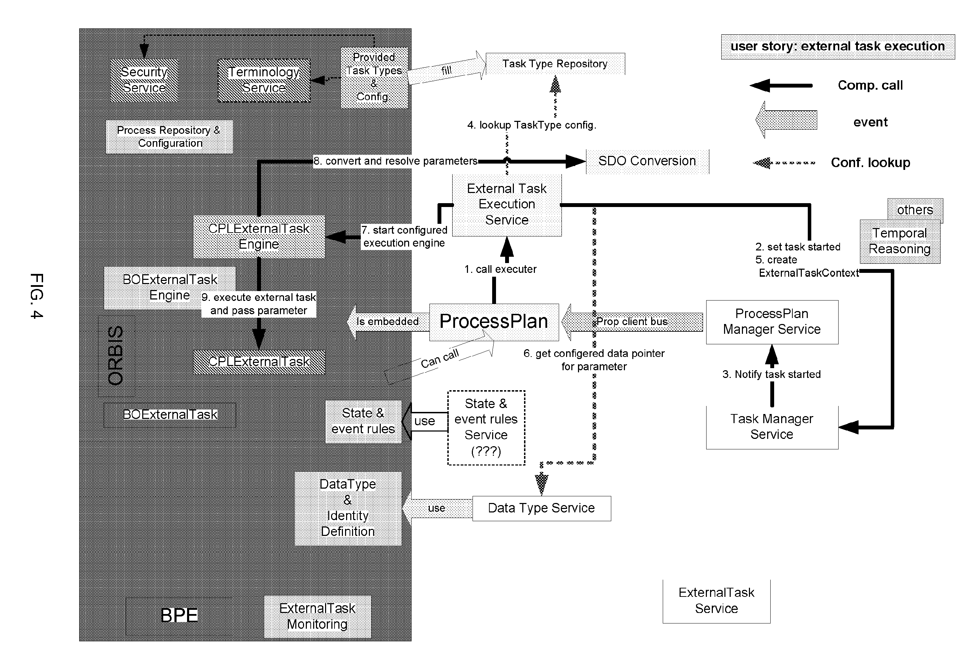

Fig. 4 is a collaboration diagram illustrating a basic task execution. Such a basic task is for example the following: a doctor is performing the execution of an element of a clinical pathway on a client computer. Upon initiating a task, a form opens and is displayed on a monitor screen and requests the user to fill in an item.

The collaboration diagram can be subdivided in two major parts: a first part running in a so-called target system or host device (in the example that is shown, the host device is part of ORBIS, a medical information system of Agfa HealthCare N.V.) and a second part relating to the client side of the core of the information system in the current architecture.

The blocks in this diagram that are important for illustration of this aspect of the invention are the following: External Task Execution Service and Task Type Repository.

A task type represents a non technical adapted activity of a target system which provides the activity. That means that the task type refers to the functional part of the activity. The technical mapping onto the activity is done by a plugged-in executer and parameter mapping defining the in and out data. With this separation a task type can be reused with another target system.

The task type repository is a virtual container for all possible task types (as connection to an external task) and the template configurations. It holds all semantic TaskType definitions as well as all technically adapted TaskTypes. It likewise holds all default parameter mappings of input and output data of the task to be executed. That means the input data is used to initiate the task and the output data is that subset of the data which the task has produced and what is sent back into the clinical path. That kind of data is in workflow systems usually called workflow relevant data.

The TaskExecution Service performs the following functions: it retrieves the Task Type and the Task data and delegates the task launch to a specialized engine. The TaskExecution Service gets the ExternalTaskContext back from execution and updates the execution state. The ExternalTaskContext consists of all data the task is producing in the execution process, the current state of the task, which can be a sub-state of an internal execution workflow, and a generic task identity object identifying the external task.

Execution of a task is performed as follows:
1. A task is selected and activated in the user interface. Hereupon the ExternalTaskExecutionService is called with a preconfiguration of the input parameter and the underlying task identification Taskid.
2. The ExternalTaskExecutionService calls the TaskManager and sets the task state (persisted) to "started".
3. The ProcessPlanManager is notified by the TaskManager about the task change. A change event is sent to the client. But the executing client ignores it since it is the event producer.
4. The technical configuration for this TaskType is looked up by accessing the repository. The task executer is determined by the correct plug-in to execute the given type of task.
5. The configuration information is used to create the transient ExternalTaskContext as runtime container for all attributes regarding the executed external task.
6. The typing of the parameter configuration is resolved against the DataTypeService.
7. The technical configuration of the engine to execute the external task is resolved and the engine is initialized.
8. The input data is resolved (converted) against the SDO (Service data object, java specification request JSR-227) type mapping and the parameters are loaded in the platform specific way.
9. The external task is initialized by the preconfigured engine and the parameters are passed into the external task.

## Claims

1. Method of executing a task part of a clinical pathway in a computer implemented medical information system **characterized in that** upon selection of said task in a user interface a technical configuration for the task type of said task is looked up in a task repository and a task executer is determined by its corresponding plug-in to execute the task.

2. Method according to claim 1 comprising the steps of
- sending a scheduling order and scheduling constraints to a scheduling device upon selection of a task to be scheduled in said user interface;
- calculating available appointment times by the scheduling device,
- communicating the available appointment times to said medical information system,
- displaying available appointment times in the user interface of said medical information system.

3. Method according to claim 2 wherein a task is marked when the scheduling has taken place.

4. Method according to claim 1 wherein in case more than one clinical pathway for a single patient are displayed in a user interface that upon dragging one task of a first pathway to a new location corresponding with a new point of time, other displayed tasks are shifted along with the dragged task, and tasks of said second pathway are not relocated.

5. Method according to claim 1 wherein when a scheduled task is dragged in the user interface to another location corresponding with another point of time, the corresponding booked appointment is released and the constraints corresponding with the new location are fed into a scheduling engine to perform a new scheduling operation.

6. A computer program product adapted to carry out the method of any of the preceding claims when run on a computer.

7. A computer readable medium comprising computer executable program code adapted to carry out the steps of any of the preceding claims.
